# EUROPEAN PATENT APPLICATION

(11) **EP 3 466 407 A1**
(43) Date of publication of application: **10.04.2019**
(21) Application number: 17801853.7
(22) Date of filing: 24.05.2017
(51) Int. Cl.: A61K 8/9789, A61Q 19/08, A61Q 19/00

(54) **COMPOSITION FOR MODULATING THE GENES RESPONSIBLE FOR GENERAL SKIN FUNCTIONS, METHOD FOR MODULATING THE EXPRESSION OF GENES RESPONSIBLE FOR GENERAL SKIN FUNCTIONS, AND USE OF A PLANT EXTRACT**

(30) Priority: 24.05.2016 BR 102016118077; 29.09.2016 BR 102016011881; 29.09.2016 BR 102016226791; 24.05.2016 BR 102016011816; 24.05.2016 BR 102016011810; 24.05.2016 BR 102016011821; 29.09.2016 BR 102016011882; 29.09.2016 BR 102016011883
(71) Applicant: Natura Cosméticos S.A., 05106-000 São Paulo SP (BR)
(72) Inventor: PAES, Fabiana, 05106-000 São Paulo - SP (BR); CAPELAS ROMEU, Clarissa, 05106-000 São Paulo - SP (BR); DE OLIVEIRA REIS, Eduardo Alexandre, 05106-000 São Paulo - SP (BR); PANZARIN SAVIETTO, Joice, 05106-000 São Paulo - SP (BR); TADINI D'ANNOLFO, Kassandra, 05106-000 São Paulo - SP (BR); CAROLLO MONCAYO, Priscila, 05106-000 São Paulo - SP (BR); SANTOS DE OLIVEIRA, Ricardo Augusto, 05106-000 São Paulo - SP (BR); EMIDIO, Simone Andrea, 05106-000 São Paulo - SP (BR); BAIONE DE MOURA, Soraya, 13208-703 Jundiaí - SP (BR); ZIMBARDI, Daniela, 05106-000 São Paulo - SP (BR)
(74) Representative: Abel & Imray
(86) International application number: PCT/BR2017/050128
(87) International publication number: WO 2017/201597

(57) **Abstract**

The present invention relates to compositions for modulating genes responsible for the general functions of the skin comprising at least one plant extract and at least one cosmetically acceptable vehicle, as well as to a method for modulating the expression of genes responsible for the general functions of the skin, and use of said plant extract in the preparation of a composition for modulating genes responsible for the general functions of the skin.

## Description

### FIELD OF THE INVENTION

The present invention relates to compositions for modulating genes responsible for the general functions of the skin comprising at least one plant extract and at least one cosmetically acceptable vehicle, as well as to a method for modulating the expression of genes responsible for the general functions of the skin, and use of said plant extract in the preparation of a composition for modulating genes responsible for the general functions of the skin.

### BACKGROUND OF THE INVENTION

Considered to be the biggest organ of a human being, the skin is the core of many complex and dynamic processes. Among these processes are barrier and immunological functions, melanine production, vitamin D synthesis, body temperature regulation, protection from ultraviolet and aesthetic radiation damage.

Good performance of the general functions of the skin may be associated with a group of genes which, once modulated, can be an interesting strategy for the development of products with comestic purposes.

However, there is a limited number of compounds that effectively act on the modulation of genes associated with general functions of the skin.

Therefore, there is a need for a cosmetic composition acting on the modulation of such genes, providing an effective treatment for skin care.

### BRIEF DESCRIPTION OF THE FIGURES

Figures 1A and 1B show an increase in involucrin protein as compared to a untreated control sample, as measured by fluorescence and percentage.
Figures 2A and 2B show an increase in ki-67 protein as compared to a untreated control sample, as measured by fluorescence and percentage.
Figures 3A and 3B show an increase in collagen protein as compared to a untreated control sample, as measured by fluorescence and percentage.
Figures 4A and 4B show an increase in elastin protein as compared to a untreated control sample, as measured by fluorescence and percentage.
Figures 5A and 5B show an increase in hyaluronic acid protein as compared to a untreated control sample, as measured by fluorescence and percentage.
Figures 6A and 6B show an increase in collagen I protein as compared to a untreated control sample, as measured by fluorescence and percentage.
Figures 7A and 7B show an increase in elastin protein as compared to a untreated control sample, as measured by fluorescence and percentage.
Figures 8A and 8B show an increase in hyaluronic acid protein as compared to a untreated control sample, as measured by fluorescence and percentage.
Figures 9A and 9B show an increase in involucrin protein as compared to a untreated control sample, as measured by fluorescence and percentage.
Figures 10A and 10B show an increase in claudin I protein as compared to a untreated control sample, as measured by fluorescence and percentage.
Figures 11A and 11B show an increase in collagen I protein as compared to a untreated control sample three (3) days after treatment, as measured by fluorescence and percentage.
Figures 12A and 12B show an increase in collagen I protein as compared to a untreated control sample 3 days after treatment, as measured by fluorescence and percentage.
Figures 13A and 13B show an increase in elastin protein as compared to a untreated control sample 3 days after treatment, as measured by fluorescence and percentage.
Figures 14A and 14B show an increase in collagen I protein as compared to a untreated control sample 3 days after treatment, as measured by fluorescence and percentage.
Figures 15A and 15B show an increase in elastin protein as compared to a untreated control sample 3 days after treatment, as measured by fluorescence and percentage.
Figures 16A and 16B show an increase in ki-67 protein as compared to a untreated control sample 3 days after treatment, as measured by fluorescence and percentage.
Figures 17A and 17B show an increase in involucrin protein as compared to a untreated control sample 3 days after treatment, as measured by fluorescence and percentage.
Figures 18A and 18B show an increase in collagen I protein as compared to a untreated control sample 3 days after treatment, as measured by fluorescence and percentage.
Figures 19A and 19B show an increase in elastin protein as compared to a untreated control sample 3 days after treatment, as measured by fluorescence and percentage.

### DESCRIPTION OF THE INVENTION

The present invention relates to a composition for modulating genes responsible for the general functions of the skin comprising at least one plant extract and at least one cosmetically acceptable vehicle.

The compositions of the present invention are suitable to be used in several age groups. Preferably, the age groups are made up of groups over 30 years of age, particularly over 45 years of age, over 60 years of age, and over 70 years of age.

For ease of reference, whenever applicable, the compositions specific to distinct age groups are designated as: compositions (30+), for ages over 30 years; compositions (45+), for ages over 45 years; compositions (60+), for ages over 60 years; and compositions (75+), for ages over 60 years.

By plant extract it is intended to mean any fraction, extract or active ingredient extracted from plants, herbs, flowers, trees, fruit, seed, roots or leaves.

Said plant is selected from the group consisting of *Acmella oleracea (spot-flower), Avena sativa, Camellia sinensis* (green tea), *Casearia sylvestris (guaçatonga (a tree of the Indian-plum family)), Cichorium intybus* (chicory), *Hymenaea courbaril* (courbaril tree), *Paeonia albiflora* (peony), *Passifloraceae* (passionflower), *Schinus terebinthifolius* (California pepper tree) and *Secale cereale,* either alone or in combination.

*Acmella oleracea,* also known as *Spilanthes acmella,* e uma erva also known as spot-flower or yellow-eye grass. It causes an anesthetic action in the buccal mucosa. The substance that is responsible for such action is an isobutylamide called spilanthol. In its chemical composition, in addition to spilanthol, there can be mentioned espilantine, aphinin, choline and phitosterin. Spilanthol, that is an active ingredient present in *Acmella oleracea,* is also sold under the tradename Spilol.

*Avena sativa,* also known as white oat, or simply oat, is a botanical species belonging to the Poaceae family. It can be obtained from the commercially available ingredient Osilift®.

*Camellia sinensis* is a species belonging to the Theaceae family, commonly known as green tea, depending on its type of cultivation. By *Camellia sinensis* it is intended to mean any fraction therefrom, particularly extracts.

*Casearia sylvestris* belongs to the Flacourtiaceae family, also known as guaçatonga. By *Casearia sylvestris* it is intended to mean any fraction therefrom, particularly extracts.

*Cichorium intybus* is a species belonging to the Compositae family, commonly known as chicory. By *Cichorium intybus* it is intended to mean any fraction therefrom, particularly extracts and actives, such as the one sold under the tradename Vederine® by Galena.

*Hymenaea courbaril* is a tree belonging to the Fabaceae family, also known as copaiba copal tree, courbaril tree or simply copaiba. Xyloglucan is an active ingredient that is present in *Hymenaea courbaril,* also known as courbaril tree xyloglucan.

*Paeonia albiflora* belongs to the Paeoniaceae family, a species of flowers. By *Paeonia albiflora* it is intended to mean any fraction therefrom, particularly extracts from their roots. Without wishing to be bound by theory, in a particular embodiment *Paeonia albiflora* can be obtained from the commercially available ingredient Volunage®, comprised by water / *Paeonia albiflora* extract / phenoxyethanol / ethylhexyl glycerin.

*Passifloraceae* is a family of angiosperms. The fruit from some species of the *Passiflora* genus are edible and known as passion fruit. Passion fruit (from the Tupi language - mara kuya, "useful fruit" or "food in the gourd") is a fruit produced by plants from the species *Passiflora edulis.* Its tree is also known as Passionflower Vine. The passionflower ceramides are of particular interest in the present invention.

*Schinus terebinthifolius* is a species belonging to the Anacardiaceae family, also known as California pepper tree ou red pepper tree. By *Schinus terebinthifolius* it is intended to mean any fraction therefrom, particularly extracts.

*Secale cereale* is a flowering plant species that belongs to the Poaceae family. Its common name is rye. *Secale cereale* can be obtained from the commercially available ingredient Coheliss®, made up of water / *Secale Cereale* extract / Penthtlene Glycol.

The composition of the present invention can HER2 comprises hyaluronic acid, caffeine and/or a mixture of sodium cocoyl amino acids and sarcosine and potassium aspartate and magnesium aspartate,

Hyaluronic acid is a biopolymer formed from hyaluronic acid and an N-acethylglucosamine. It is a glucosaminoglycan that can be obtained either from a natural or synthetic source.

By caffeine it is also intended to mean any raw-material containing caffeine, in particular that sold under the tradename Ecoslim® by Lucas Meyer, which is made up of a green tea extract containing caffeine as an active ingredient.

The mixture of sodium cocoyl amino acids and sarcosine and potassium aspartate and magnesium aspartate is sold under the tradename Sepicalm S by Seppic.

To make up the compositions of the present invention, particularly preferred are extracts or active ingredients extracted from *Camellia sinensis, Casearia sylvestris, Schinus terebinthifolius, Paeonia albiflora, Cichorium intybus, Hymenaea courbaril, Avena sativa, Secale cereale;* passionflower ceramides; the active ingredients spilanthol and xyloglucan extracted from *Acmella oleracea* and *Hymenaea courbaril,* respectively; hyaluronic acid, caffeine, a mixture of sodium cocoyl amino acids and sarcosine and potassium aspartate and magnesium aspartate; either alone or in combination.

Individually, the therapeutic and cosmetic effects of the above mentioned components have been a subject of study.

However, Applicant has surprisingly found that by formulating the above mentioned components into a composition, those components have a modulation effect on genes responsible for the general functions of the skin.

Preferred compositions of the present invention comprise the following combinations:
- *Camellia sinensis* extract and spilanthol - are preferably compositions (30+);
- courbaril tree xyloglucan and a mixture of sodium cocoyl amino acids and sarcosine and potassium aspartate and magnesium aspartate - are preferably compositions (45+);
- *Casearia sylvestris, Schinus terebinthifolius,* and *Paeonia albiflora* extracts - are preferably compositions (60+);
- passionflower ceramides and *Cichorium intybus* extract - are preferably compositions (70+);
- spilanthol and hyaluronic acid - are preferably compositions in the form of an elixir;
- *Hymenaea courbaril, Paeonia albiflora, Secale cereale* and *Avena sativa* extracts - are preferably compositions in the form of serum;
- *Casearia sylvestris, Schinus terebinthfolius* extracts and hyaluronic acid - are preferably compositions in the form of a filler; and
- *Schinus terebinthfolius* extract and caffeine - are preferably compositions in the form of gel.

Such genes include at least one of the CAT, GPX1, MSRA, NOX1, PRDX6, SOD2, DSC2, IGF1R, ITGA1, ITGB1, LAMB1, LAMB3, DEFB4A, CDH1, CAMP, CLDN1, CLDN4, CLDN7, CDSN, DSG4, DSP, ELOVL3, GBA, ITGA6, ITGB4, KRT19, KRT10. KRT14, KRT16, KRT17, KRT1, KRT6A, OR2AT4, LAMA5, OCLN, PKP1, PLEC, TGM5, TGM1, RNASE7, SMPD1, SDC1, TJP1, VCL, BTC, FLT1, PDGFRA, HAS1, HAS2, SIRT1, SRD5A1, PRLR, HSD17B2, TPH1, AANAT, ASMT, MTNR1A, AR, ESR2, CYP19A1, HTR2A, HTR2B, MTOR, AQP3, CTSB, CTSE, CTSL, CD44, HAL, PADI3, PADI1, CASP14, FLG, ST14, IL13, IL18, IL19, ITGA2, IL1A, IL1B, IL22, IL17A, IL1R1, IL10. IL6, F2RL1, TRPV1, CALCA, ACACA, ASAH1, UGCG, SPTLC1, SREBF2, GLB1, ADAM9, MMP1, MMP10. MMP2, MMP3, MMP9, SERPINE1, TIMP1, TIMP2, TMPRSS6, FBLN5, FBN1, MMP12, MMP13, MMP14, TYR, GPNMB, MAP1LC3B, POMC, OPRM1, MC1R, MITF, CANX, HSPB1, HSPA1A, EGFR, TGFB1, TGFBR1, NGF, PDGFA, VEGFA, VEGFB, FGFR1, FGFR2, FGF1, FGF2, MMP11, HYAL1, HYAL2, ELN, LOX, COL1A1, COL1A2, ACO2, ANXA5 genes.

More surprisingly, Applicant has found that:
- In amounts of about 0.025% of *Camellia sinensis* and about 0.125% of spilanthol, a synergistic modulation occurs in such genes, particularly in modulating the expression of at least one of the involucrin and ki-67 proteins;
- In amounts of about 0.25% of courbaril tree xyloglucan and about 1.5% of a mixture of sodium cocoyl amino acids and sarcosine and potassium aspartate and magnesium aspartate, a synergistic modulation occurs in such genes, particularly in modulating the expression of collagen, elastin proteina and/or expression of hyaluronic acid protein, providing its use in cosmetic applications in the skin in general;
- In amounts of about 0.05% of *Casearia sylvestris,* about 0.0125 de *Schinus terebinthifolius* and about 2% *Paeonia albiflora,* a synergistic modulation occurs in such genes, particularly in modulating the expression of at least one of the collagen, elastin and hyaluronic acid proteins;

- In amounts of about 0.3% of passionflower ceramides and about 3% of *Cichorium intybus,* a synergistic modulation occurs in such genes, particularly in modulating the expression of the involucrin protein and claudin I protein, providing its use in cosmetic applications in the skin in general;
- In amounts of about 0.25% of spilanthol and about 5% of hyaluronic acid, a synergistic modulation occurs in such genes, particularly in modulating the expression of the collagen I protein, providing its use in cosmetic applications in the skin in general;
- In amounts of about 0.5% *Hymenaea courbaril,* about 2% of *Paeonia albiflora,* about 4% of *Secale cereale* and about 4% of *Avena sativa,* a synergistic modulation occurs in such genes, particularly in modulating the expression of the collagen I protein and elastin protein, providing its use in cosmetic applications in the skin in general;
- In amounts of about 0.1% of *Casearia sylvestris,* about 0.025% of *Schinus terebinthfolius* and about 5% of hyaluronic acid, a synergistic modulation occurs in such genes, particularly in modulating the expression of the collagen I, elastin, ki-67 and involucrin proteins, providing its use in cosmetic applications in the skin in general; and
- In amounts of about 0.35% of *Schinus terebinthfolius* and about 1% of caffeine or a raw-material containing caffeine, a synergistic modulation occurs in such genes, particularly in modulating the expression of the collagen I protein and elastin protein, providing its use in cosmetic applications in the skin in general.

Accordingly, the compositions according to the present invention efficiently act on cell differentiation and proliferation, providing its use in cosmetic applications in the skin in general.

The compositions of the present invention are preferably anti-sign cosmetic compositions and can be in the form of gel, gel cream, elixir, serum, *inter alia,* as known by those skilled the art from the used cosmetic vehicles.

The present invention also relates to a method for modulating the expression of such genes responsible for the general functions of the skin, which method comprises the step of administering a composition according to the present invention to an individual skin.

As used herein, by skin it is intended to mean neck, face, arm, forearm, chest, and hand skin.

The present invention further relates to the use of at least one plant extract in the preparation of a composition for modulating genes responsible for the general functions of the skin.

The present invention relates, in particular, to the use of the following combinations in the preparation of a composition:
- *Camellia sinensis* extract and spilanthol;
- courbaril tree xyloglucan and a mixture of sodium cocoyl amino acids and sarcosine and potassium aspartate and magnesium aspartate;
- *Casearia sylvestris, Schinus terebinthifolius* and *Paeonia albiflo*ra extracts;
- passionflower ceramides and *Cichorium intybus* extract;
- spilanthol and hyaluronic acid;
- *Hymenaea courbaril, Paeonia albiflora, Secale cereale* and *Avena sativa* extracts;
- *Casearia sylvestris, Schinus terebinthfolius* extracts and hyaluronic acid; and
- *Schinus terebinthfolius* extract and caffeine.

The genes according to the present invention and their correlation with the general functions of the skin are defined in the following table.

**Table 1. Definition of genes and their correlation with the general function of the skin.**

| **Gene name** | **Symbol** | **General function** |
|---|---|---|
| Catalase | CAT | Antioxidant defense |
| Glutathione peroxidase 1 | GPX1 | Antioxidant defense |
| methionine sulfoxide reductase A | MSRA | Antioxidant defense |
| NADPH oxidase 1 | NOX1 | Antioxidant defense |
| peroxiredoxin 6 | PRDX6 | Antioxidant defense |
| Superoxide dismutase 2, mitochondrial | SOD2 | Antioxidant defense |
| desmocollin 2 | DSC2 | Adhesion / anchorage |
| Insulin like growth factor 1 receptor | IGF1R | Adhesion / anchorage |
| integrin, alpha 1 | ITGA1 | Adhesion / anchorage |
| integrin, beta1 | ITGB1 | Adhesion / anchorage |
| laminin, beta 1 | LAMB1 | Adhesion / anchorage |
| laminin, beta 3 | LAMB3 | Adhesion / anchorage |
| b-defensin 2, beta 4 | DEFB4A | Bactericidal activity |
| Cadherin-1 | CDH1 | Barrier function / cohesion |
| Cathelicidin antimicrobial peptide | CAMP | Barrier function / cohesion |
| Claudin-1 | CLDN1 | Barrier function / cohesion |
| Claudin-4 | CLDN4 | Barrier function / cohesion |
| Claudin-7 | CLDN7 | Barrier function / cohesion |
| Corneodesmosin | CDSN | Barrier function / cohesion |
| Desmoglein-4 | DSG4 | Barrier function / cohesion |
| Desmoplakin | DSP | Barrier function /cohesion |
| elongation of very long chain fatty acids (FEN1/Elo2, SUR4/Elo3, yeast)-like 3 | ELOVL3 | Barrier function / cohesion |
| Glucosylceramidase | GBA | Barrier function / cohesion |
| Integrin alpha-6 | ITGA6 | Barrier function / cohesion |
| Integrin beta-4 | ITGB4 | Barrier function / cohesion |
| Keratin 19 | KRT19 | Barrier function / cohesion |
| Keratin-10 | KRT10 | Barrier function / cohesion |
| Keratin-14 | KRT14 | Barrier function / Cohesion |
| Keratin-16 | KRT16 | Barrier function / Cohesion |
| Keratin-17 | KRT17 | Barrier function / Cohesion |
| Keratin-1 | KRT1 | Barrier function / cohesion |
| Keratin-6 | KRT6A | Barrier function / Cohesion |
| Olfactory receptor, family 2, subfamily AT, member 4 | OR2AT4 | Wound healing |
| Laminin subunit alpha-5 | LAMA5 | Barrier function / Cohesion |
| Occludin | OCLN | Barrier function / Cohesion |
| Plakophilin 1 | PKP1 | Barrier function / cohesion |
| Plectin-1 | PLEC | Barrier function / Cohesion |
| Transglutaminase-5 | TGM5 | Barrier function / cohesion |
| Transglutaminase 1 | TGM1 | Barrier function / cohesion |
| RNase A family, 7 | RNASE7 | Barrier function / cohesion |
| Acid sphingomyelinase | SMPD1 | Barrier function / cohesion |
| Syndecan 1 | SDC1 | Barrier function / cohesion |
| Tight junction protein 1 | TJP1 | Barrier function / cohesion |
| Vinculin | VCL | Barrier function / cohesion |
| Betacellulin | BTC | Cell growth |
| Vascular permeability factor receptor | FLT1 | Cell growth |
| Platelet-derived growth factor (PDGF) receptor A | PDGFRA | Cell proliferation |
| Hyaluronan synthase 1 | HAS1 | Cell matrix adhesion |
| Hyaluronan synthase 2 | HAS2 | Cell matrix adhesion |
| Sirtuin 1 | SIRT1 | histone deacetylase |
| Steroid-5-alpha- reductase | SRD5A1 | Hormone metabolism |
| Prolactin receptor | PRLR | Hormone metabolism |
| Hydroxysteroid (17-beta) dehydrogenase 2 | HSD17B2 | Hormone metabolism |
| Tryptophan hydroxilase 1 | TPH1 | Hormone metabolism |
| Aralkylamine N-acetyltransferase | AANAT | Hormone metabolism |
| Hydroxyindole O-methyltransferase | ASMT | Hormone metabolism |
| Melatonin receptor 1^{a} | MTNR1A | Hormone metabolism |
| Androgen receptor | AR | Hormone metabolism |
| Estrogen receptor | ESR2 | Hormone metabolism |
| Cytochrome P450. family 19, subfamily A, polypeptide 1 / Aromatase | CYP19A1 | Hormone metabolism |
| 5-hydroxytryptamine (serotonin) receptor 2^{a} | HTR2A | Hormone metabolism |
| 5-hydroxytryptamine (serotonin) receptor 2B | HTR2B | Hormone metabolism |
| Mechanistic target of rapamycin | MTOR | |
| Aquaporin-3 | AQP3 | Hydration |
| Cathepsin B | CTSB | Hydration |
| Cathepsin E | CTSE | Hydration |
| Cathepsin L1 | CTSL | Hydration |
| CD44 antigen | CD44 | Hydration |
| Histidine ammonia-lyase | HAL | Hydration |
| Protein-arginine deiminase type-3 | PADI3 | Hydration |
| Peptidyl-arginine deiminase, type I | PADI1 | Hydration |
| Caspase-14 | CASP14 | Hydration/differentiation/barrier formation |
| Filaggrin | FLG | Hydration/differentiation/barrier formation |
| Serine protease 14/Matriptase | ST14 | Hydration/differentiation/barrier formation |
| Interleukin-13 | IL13 | Immune response |
| Interleukin-18 | IL18 | Immune response |
| Interleukin-19 | IL19 | Immune response |
| Integrin, alpha 2 | ITGA2 | linflammation |
| Interleukin 1, alpha | IL1A | linflammation |
| Interleukin 1, beta | IL1B | linflammation |
| Interleukin 22 | IL22 | linflammation |
| Interleukin 17^{a} | IL17A | linflammation |
| Interleukin-1 receptor type I | IL1R1 | linflammation |
| Interleukin-10 | IL10 | linflammation |
| interleukin 6 | IL6 | linflammation |
| Protease-activated receptor 2 (kal-likrein receptor) | F2RL1 | Peeling |
| Activation of vanilloid receptor-1 | TRPV1 | Itching |
| Calcitonin gene-related peptide | CALCA | Itching |
| Acetyl-CoA carboxylase 1 | ACACA | Synthesis of lipid |
| Acid ceramidase | ASAH1 | Synthesis of lipid |
| Ceramide glucosyltransferase | UGCG | Synthesis of lipid |
| Serine palmitoyltransferase 1 | SPTLC1 | Synthesis of lipid |
| Sterol regulatory element-binding protein 2 | SREBF2 | Synthesis of lipid |
| Galactosidase, beta 1 | GLB1 | Liposomal hydrolase |
| ADAM metallopeptidase domain 9 | ADAM9 | Matrix remodeling |
| Matrix metallopeptidase 1 (interstitial collagenase) | MMP1 | Matrix remodeling |
| Matrix metallopeptidase 10 (strome-lysin 2) | MMP10 | Matrix remodeling |
| Matrix metallopeptidase 2 (gelatinase A, type IV collagenase) | MMP2 | Matrix remodeling |
| Matrix metallopeptidase 3 (strome-lysin 1, progelatinase) | MMP3 | Matrix remodeling |
| Matrix metallopeptidase 9 (gelatinase B, type IV collagenase) | MMP9 | Matrix remodeling |
| Serpin peptidase inhibitor | SERPINE1 | Matrix remodeling |
| TIMP metallopeptidase inhibitor 1 | TIMP1 | Matrix remodeling |
| TIMP metallopeptidase inhibitor 2 | TIMP2 | Matrix remodeling |
| Transmembrane protease, serine 6 | TMPRSS6 | Matrix remodeling |
| Fibulin 5 | FBLN5 | Matrix remodeling |
| Fibrilin 1 | FBN1 | Matrix remodeling |
| Matrix metallopeptidase 12 | MMP12 | Matrix remodeling/ wound healing |
| Matrix metallopeptidase 13 | MMP13 | Matrix remodeling/wound healing |
| Matrix metallopeptidase 14 | MMP14 | Matrix remodeling/ wound healing |
| Tyrosinase | TYR | Synthesis of melanine |
| Glycoprotein (transmembrane) nmb | GPNMB | Adhesion of melanocytes to keratinocytes |
| MAP1LC3B microtubule-associated protein 1 light chain 3 beta | MAP1LC3B | Autophagy |
| Proopiomelanocortin | POMC | Neuropeptide metabolism |
| Opioid receptor | OPRM1 | Neuropeptide metabolism |
| Melanocortin 1 receptor | MC1R | Neuropeptide metabolism |
| Microphthalmia-associated transcription factor | MITF | Melanocyte regulator |
| Calnexin | CANX | Protein folding |
| Heat shock 27kDa | HSPB1 | Protein folding |
| Heat shock 72kDa | HSPA1A | Protein folding |
| Epidermal growth factor receptor | EGFR | Signal transduction |
| Transforming growth factor, beta 1 | TGFB1 | Signal transduction |
| Transforming growth factor, beta | TGFBR1 | Signal transduction |
| receptor I | | |
| Nerve Growth Factor | NGF | Wound healing |
| Platelet-derived Growth Factor A | PDGFA | Wound healing |
| Vascular Endothelial Growth Factor A | VEGFA | Wound healing |
| Vascular Endothelial Growth Factor B | VEGFB | Wound healing |
| Fibroblast growth factor receptor 1 | FGFR1 | Wound healing |
| Fibroblast growth factor receptor 2 | FGFR2 | Wound healing |
| Fibroblast growth factor 1 (acidic) | FGF1 | Wound healing |
| Fibroblast growth factor 2 (basic) | FGF2 | Wound healing |
| Matrix metallopeptidase 11 | MMP11 | Wound healing |
| Hyaluronidase 1 | HYAL1 | Hydration |
| Hyaluronidase 2 | HYAL2 | Hydration |
| Elastin | ELN | Matrix remodeling |
| Lysyl oxidase | LOX | Matrix remodeling |
| Collagen, type I, alpha 1 | COL1A1 | Matrix remodeling |
| Collagen, type I, alpha 2 | COL1A2 | Matrix remodeling |
| Aconitase 2, mitochondrial | ACO2 | Antioxidant defense |
| Annexin A5 | ANXA5 | Cell growth/differentiation |

Cosmetically acceptable vehicles according to the present invention include, but without any limitation, those known in the art.

As non-limitative examples there can be mentioned: preservatives, perfumes/fragrances, polymer neutralizing agents, chelating agents, pH adjustment agents, and the like. Particularly used are disodium EDTA dissódi-co (chelating agent), iodopropynyl butylcarbamate (preservative), phenoxyethanol (preservative), wild basil essential oil (perfume) and triethanolamine (pH adjusting agent).

The following examples, but not limited thereto, illustrate the present invention, particularly with regard to the effects on the modulation of genes associates with the general functions of the skin.

### Example 1. Preparation of cosmetic compositions

Cosmetic compositions were prepared in the form of oil-in-water gel-cream emulsions, in which the oil is the dispersed phase and water is the continuous phase. Both phases were heated at a temperature from 75-80°C, thereafter the oil phase, containing the sunscreens, was poured into the aqueous phase with stirring for about 10 minute. Subsequently, the process cooling step was started by adding an aqueous phase containing a polymer neutralizing agent. When the temperature reached about 60°C, the phase containing sensorial modifiers (silicones) was added thereto and when the temperature reached 40°C, the preservatives, fragrance, sensorial modifiers (particles) and high temperature-sensitive actives were added thereto.

### Example 2. Preparation of cosmetic compositions

Cosmetic compositions were prepared in the form of oli-in-water gel-cream emulsions, in which the oil is the dispersed phase and water is the continuous phase. In this case, heating of the aqueous phase was started within the main vessel up to a temperature of about 75 and about 80°C and, with stirring, the oil phase was added thereto with stirring for about 10 minutes. Subsequently, the process cooling step was started by adding an aqueous phase containing a polymer neutralizing agent. When the temperature reached about 60°C, the phase containing sensorial modifiers (silicones) was added thereto and when the temperature reached 40°C, the preservatives, fragrance, sensorial modifiers (particles) and high temperature-sensitive actives were added thereto.

### Example 3. Molecular activity assay

A large scale gene expression profile assay (by PCR array) was performed of 180 genes in skin explants obtained subsequent to blepharo-plasty and subjected to individual treatments with the present invention.

Initially, skin explants were obtained from the eyelids of females between 45 and 55 years of age, three (3) different donors of each age. The explants were split into halves and immediately inserted, in triplicate from each donor, into a culture medium and kept for 24 hours (half 1) and 72 hours (half 2) in a wet environment, at 37°C, 5% CO₂. During this time the explants were subjected to treatments with 2mg/cm² of each sample (compositions according to the invention) topically applied on the explants, without being diluted. Furthermore, a control was used by maintaining the explants, in triplicate from each donor, in a culture medium alone. A prior analysis of viability was performed to make sure the tissues would be preserved during the study protocol for all formulae investigated.

Half (1) was collected and subjected to total RNA extraction. The quality of the extracted RNA was qualitatively (*Bioanalyzer microcapillary electrophoresis*) and quantitatively (*Nanodrop spectrophotometer*) assessed. From the RNA, the cDNA was engineered and subject to a real-time RT-PCR (*Polymerase* - *Chain Reaction*) step to evaluate the expression of 180 genes by using the customized platform Taqman PCR Array (ThermoFisher). The test genes were those enumerated in table 1 by using the StepOne Plus (Life Technologies) equipment. The gene expression profile and selection of the differently expressed genes were carried out by using the Expression Suite Software v.1.0.3 (Life Technologies). The ΔCt values for the GAPDH (glyceraldehyde-3-phosphate dehydrogenase) reference gene and target gene were calculated by subtracting from each other the values for the experimental groups. Subsequently, ΔCt for the experimental group was subtracted from the control group (untreated skin explant) for obtaining ΔΔCt. Lastly, a relative quantification of the target genes was determined by using the equation: RQ = 2-ΔΔCt. Only those genes exhibiting a threshold of 1,3, that is, a 30% increase or reduction as compared to the control, were selected. The statistical significance was assessed by using the t test followed by the Benjamini-Hochberg method (FDR - false discovery rate), which p-value < 0.05 was considered significant. Those detected values considered as statistically significant were entered in the software Ingenuity Pathway Analysis (IPA) (http://www.ingenuity.com), in order to investigate the functional relationships between those identified genes. For each established pathway/network a um p-score [p-score = -log10 (p-value)] was generated reflecting the probability of such a randomly generated network and wherein the p-value was calculated by using the Fisher's exact test. This means that if a pathway has a p-score de 10, the odds of such pathway being randomly generated is less than 1 in 1010. These results show the genes and their functions are modulated by the compositions and methods of the present invention.

Half (2) was collected, fixed on para-formaldehyde 4% (pH 7.4) for 24 hours and cryoprotected in a 30% sucrose solution for 48 hours. Then, 10 µm serial slices were directly collected on silanized slides by using a Cryostat (Leica - CN1850). Once the slice collection is complete, the slices were washed with 0.1 M PB and incubated overnight with antibodies relating with the selected proteins of interest. Then, the slides were analyzed under a Fluorescence Microscope (Leica - DM 1000) by using the LAS (Leica Application Suite) Software. Fluorescence intensity emitted by a antibody-specific marker was the parameter that was evaluated. For the statistical analysis variance analysis (ANOVA) was used. In all the groups that have been studied, considered to be statistically significant were those groups which P values were inferior to 0.05.

### Composition 1 (30+)

The untreated control, an active ingredient-free cosmetic-based composition, a cosmetic-based composition containing 0.025% of *Camellia sinensis,* a cosmetic-based composition containing 0.125% of spilanthol and an identical cosmetic base containing 0.025% of *Camellia sinensis* and 0.125% of spilanthol were investigated.

As to the gene expression, as compared to the untreated control, it was seen that the composition comprising *Camellia sinensis* was able to modulate 95 genes, the composition comprising spilanthol was able to modulate 101 genes and the composition comprising a combination of 0.025% of *Camellia sinensis* and 0.125% of spilanthol was able to modulate 110 genes. The main identified mechanisms include the endogenous antioxidant system stimulation, adhesion molecules (desmosomes) stimulation, anti-inflammatory action, elastic fiber stimulation, horned envelope formation / barrier reinforcement and collagen stimulation.

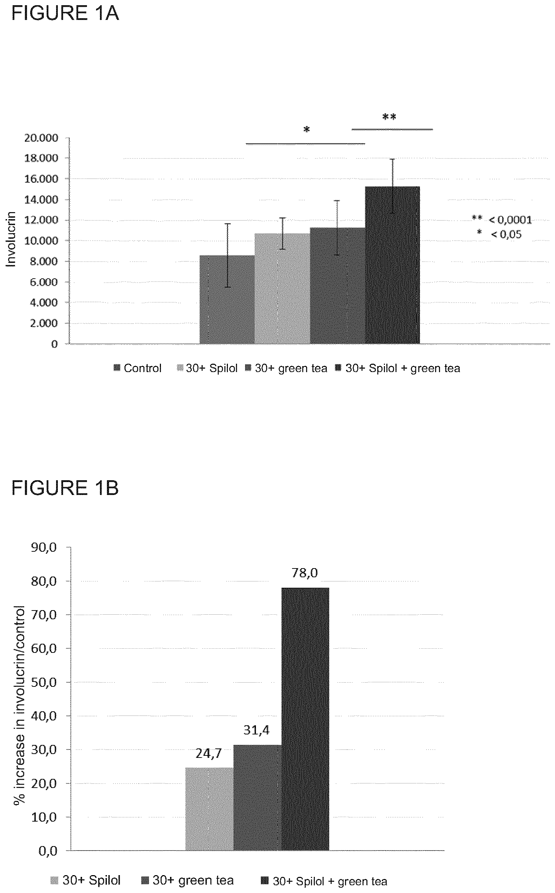

With respect to protein expression, a synergistic effect has been seen for the combination of spilanthol and *Camellia sinensis* on the involucrin protein (cell differentiation marker), since the composition comprising *Camellia sinensis* (green tea) and the composition comprising spilanthol promoted a 24,7% and 31,4% increase, respectively, while the composition comprising the combination promoted a 78% increase in this protein expression (figures 1A and 1B).

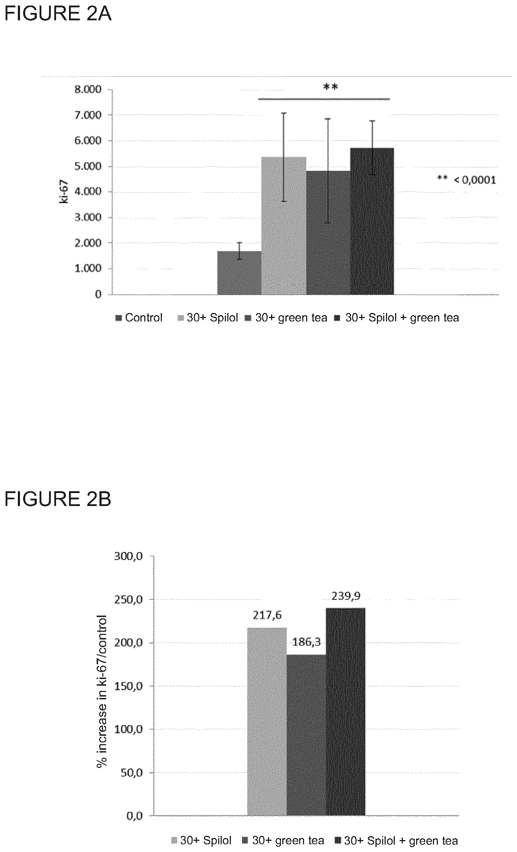

Furthermore, the combination of spilanthol and *Camellia sinensis* promoted a 240% increase in the expression of the **ki-67** protein, which protein is a marker for cell proliferation (figures 2A and 2B).

### Composition 2 (45+)

The untreated control, an active ingredient-free cosmetic-based composition, a cosmetic-based composition containing 0.25% of courbaril tree xyloglucan, a cosmetic-based composition containing 1.5% of Sepicalm and an identical cosmetic base containing 0.25% of courbaril tree xyloglucan and 1.5% of Sepicalm were investigated.

As to the gene expression, as compared to the untreated control, it was seen that the courbaril tree xyloglucan sample was able to modulate 114 genes, the Sepicalm sample was able to modulate 128 genes and the combination of courbaril tree xyloglucan and Sepicalm was able to modulate 153 genes. Firmness, elastic fiber stimulation, cell differentiation, filling, hydration, anti-inflammatory, dermis-epidermis cohesion, and horned envelope formation / barrier reinforcement mechanisms were the main identified mechanisms.

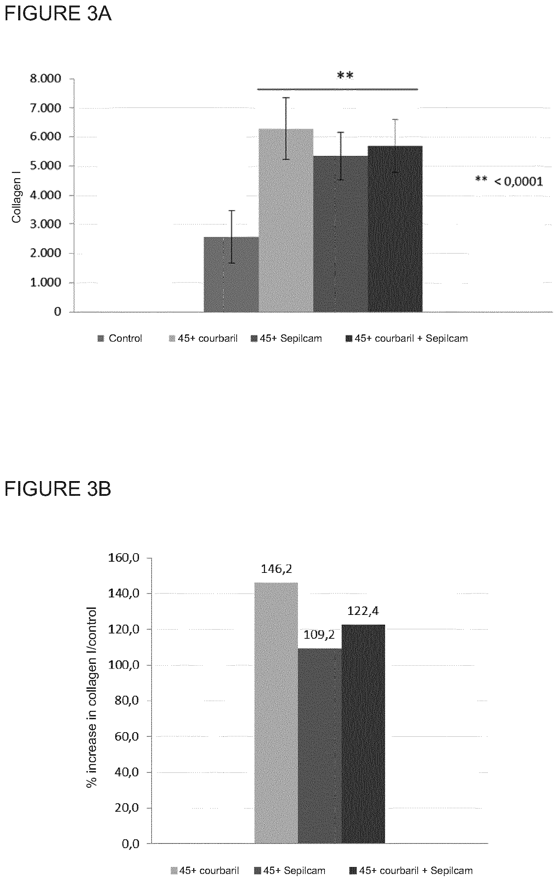

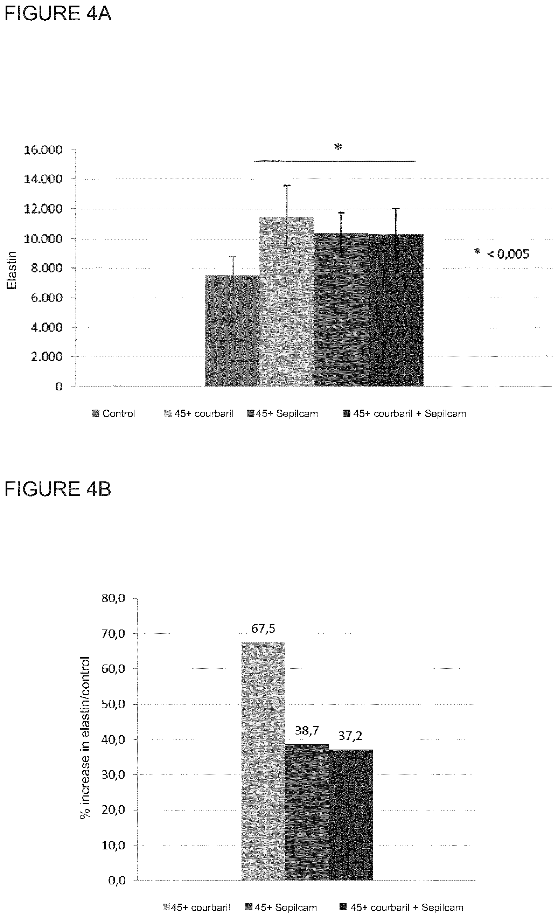

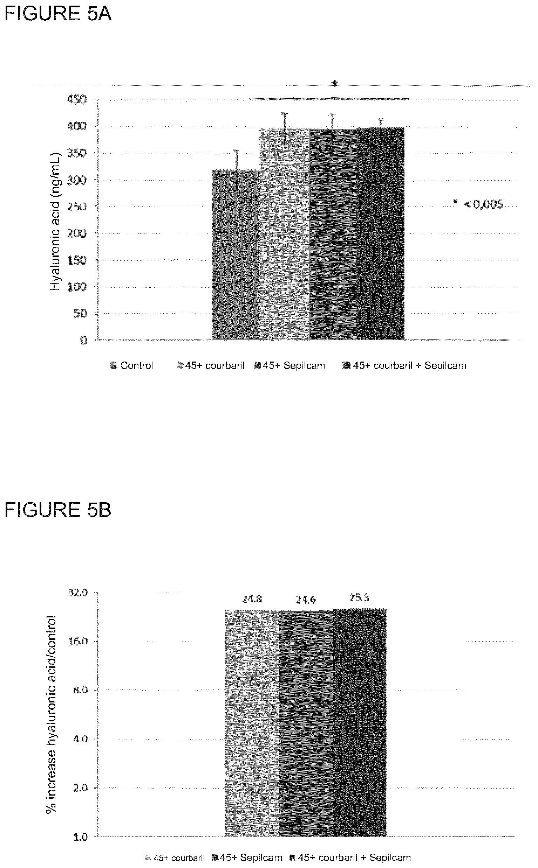

With respect to protein expression, the combination of courbaril tree xyloglucan and Sepicalm promoted a 122,4% increase in the expression of the collagen protein (Figures 3A and 3B), a 37,2% increase in the expression of the elastin protein (Figures 4A and 4B) and a 25,3% increase in the expression of the hyaluronic acid protein (Figures 5A and 5B).

### Composition 3 (60+)

The untreated control, an active ingredient-free cosmetic-based composition, a cosmetic-based composition containing 0.05% of *Casearia sylvestris* and about 0.0125 of *Schinus terebinthifolius,* a cosmetic-based composition containing 2% of *Paeonia albiflora* and an identical cosmetic base containing 0.05% of *Casearia sylvestris,* 0.0125% of *Schinus terebinthifolius* and 2% of *Paeonia albiflora* were investigated.

With respect to gene expression, as compared to the untreated control, it was seen that the guaçatonga extract + California pepper tree extract sample was able to modulate 151 genes, the Volunage sample was able to modulate 117 genes and the combination of guaçatonga extract + California pepper tree extract + volunage was able to modulate 162 genes. Firmness, elastic fiber stimulation, filling, hydration, anti-inflammatory, dermis-epidermis cohesion, horned envelope formation, and barrier reinforcement mechanisms were the main identified mechanisms.

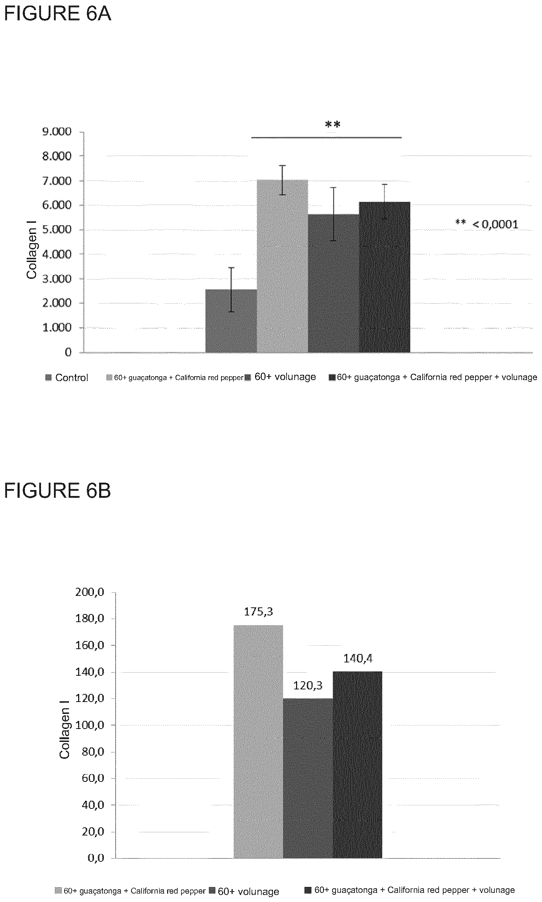

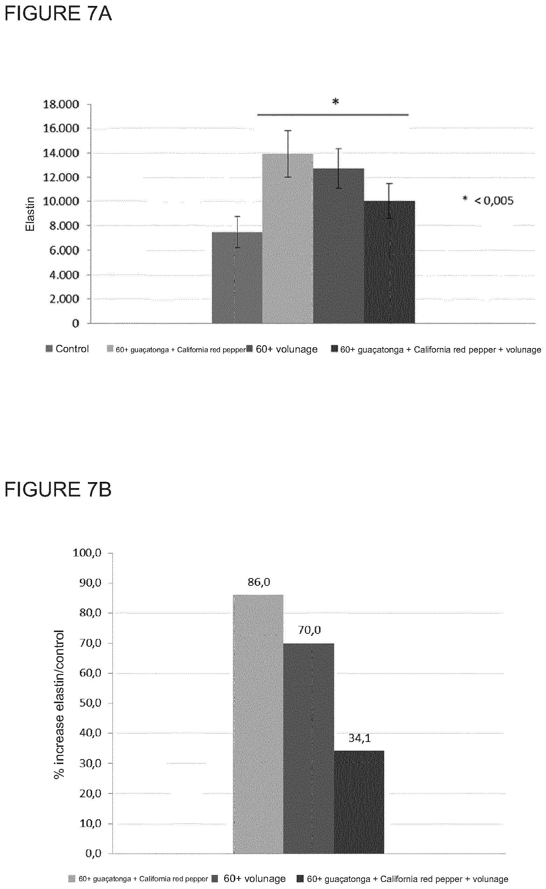

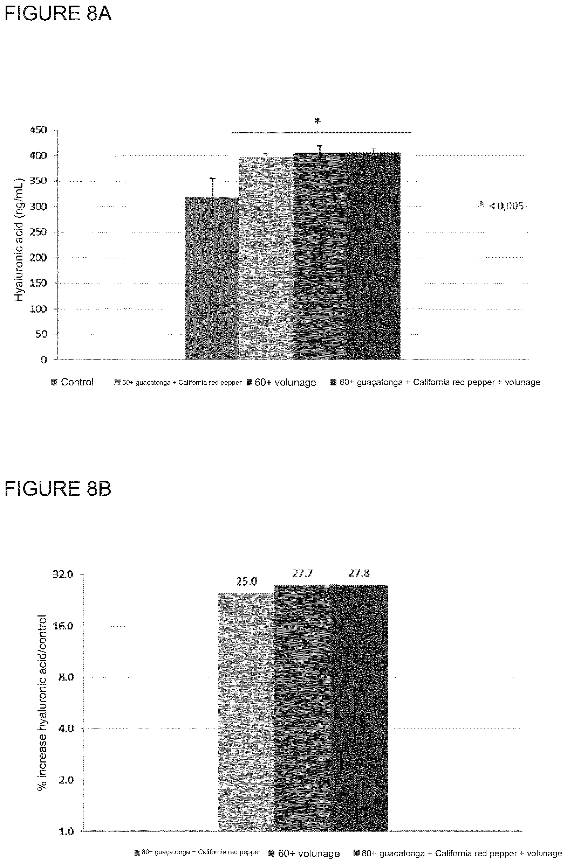

With respect to protein expression, the combination of guaçatonga extract and California pepper tree extract and Volunage promoted a 140.4% increase in the expression of the collagen I protein (figure 6A and 6B), a 34,1% increase in the expression of the elastin protein (figure 7A and 7B) and a 27,8% increase in the expression of the hyaluronic acid protein (figure 8A and 8B).

### Composition 4 (70+)

The untreated control, an active ingredient-free cosmetic-based composition, a cosmetic-based composition containing 0.3% of passionflower ceramides, a cosmetic-based composition containing 3% of Vederine and an identical cosmetic base containing 0.3% of passionflower ceramides and 3% of Vederine were investigated.

As to the gene expression, as compared to the untreated control, it has been seen that the passionflower ceramide sample was able to modulate 148 genes, the Vederine sample was able to modulate 151 genes and the combination of passionflower ceramide and Vederine was able to modulate 146 genes.

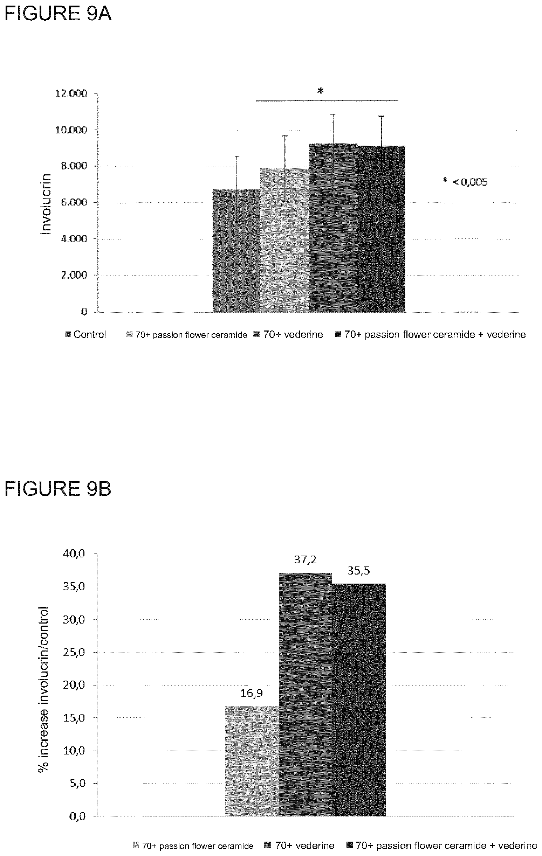

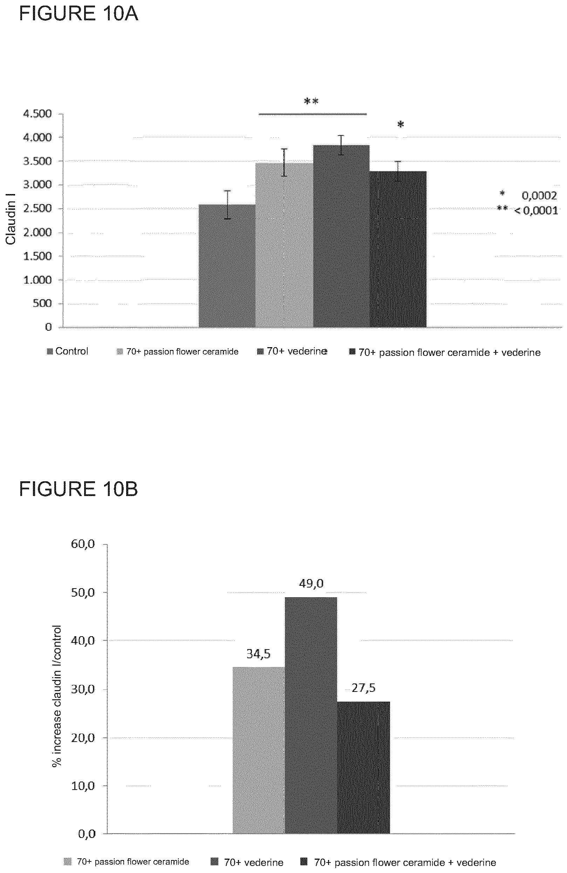

As to the protein expression, the combination of passionflower ceramide and Vederine promoted a 35,5% increase in the expression of the involucrin protein (Figures 9A and 9B) and a 27,5% increase in the expression of the claudin I protein (Figure 10A and 10B).

### Composition 5 (elixir)

The untreated control, placebo, a cosmetic composition containing the combination of spilanthol and hyaluronic acid (wrinkle reducer elixir), 0.25% spilanthol, 5% hyaluronic acid and the combination of 0.25% spilanthol and 5% hyaluronic acid were investigated.

As to the gene expression, as compared to the untreated control, it was seen that the spilanthol sample was able to modulate 163 genes, the Hyaluronic Acid sample was able to modulate 146 genes and the combination of spilanthol and Hyaluronic Acid was able to modulate 149 genes. As compared to the untreated control, a one-fold effect relative to collagen I (firmness mechanism), hyaluronic acid (filling mechanism), claudin I (cell-cell communication mechanism), two-fold relative to elastin (elastic fiber stimulation), involucrin (cell differentiation mechanism), ki-67 (cell proliferation - renewal mechanism), SOD2 (antioxidant mechanism), B1 or B4 integrin (dermis-epidermis cohesion mechanism) and five-fold relative to IL-10 (anti-inflammatory mechanism) was seen.

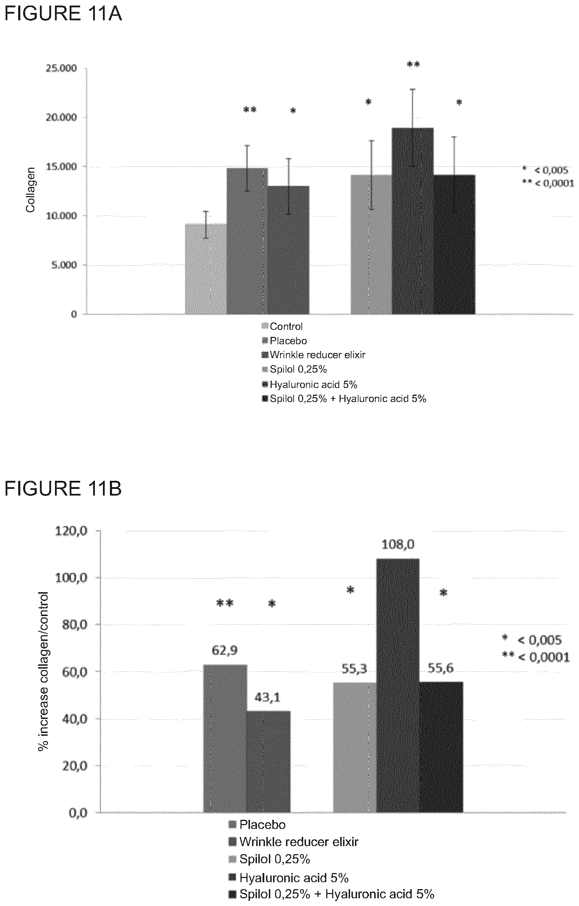

As to the protein expression, the combination of spilanthol and Hyaluronic Acid promoted a 56% increase in the expression of the collagen I protein (Figures 11A and 11B).

### Composition 6 (serum)

The untreated control, placebo, a cosmetic composition containing a combination of *Hymenaea courbaril, Paeonia albiflora, Secale cereale* and *Avena sativa* (serum firmness), *Hymenaea courbaril* (Courbaril tree 0.5%), *Paeonia albiflora, Secale cereale* and *Avena sativa* (2% Volunage + 4% Osilift + 4% Coheliss) and a combination of *Hymenaea courbaril, Paeonia albiflora, Secale cereale* and *Avena sativa* (0.5% Courbaril tree + 2% Volunage + 4% Osilift + 4% Coheliss) were investigated.

As to the gene expression, as compared to the untreated control, it was seen that the 0.5% Courbaril tree sample was able to modulate 147 genes, the 2% Volunage sample + 4% Osilift + Coheliss 4 was able to modulate 144 genes and the combination of 0.5% Courbaril tree + 2% Volunage + 4% Osilift + 4% Coheliss was able to modulate 121 genes. The combination according to the present invention has been seen to exhibit a one-fold effect as compared to the untreated control on collagen (firmness mechanism), hyaluronic acid (filling mechanism) and B1 or B4 integrin (dermis-epidermis cohesion mechanism), as well a two-fold effect as compared to the untreated elastin control (elastic fiber stimulation mechanism), involucrin (cell differentiation mechanism), SOD2 (endogenous antioxidant system mechanism).

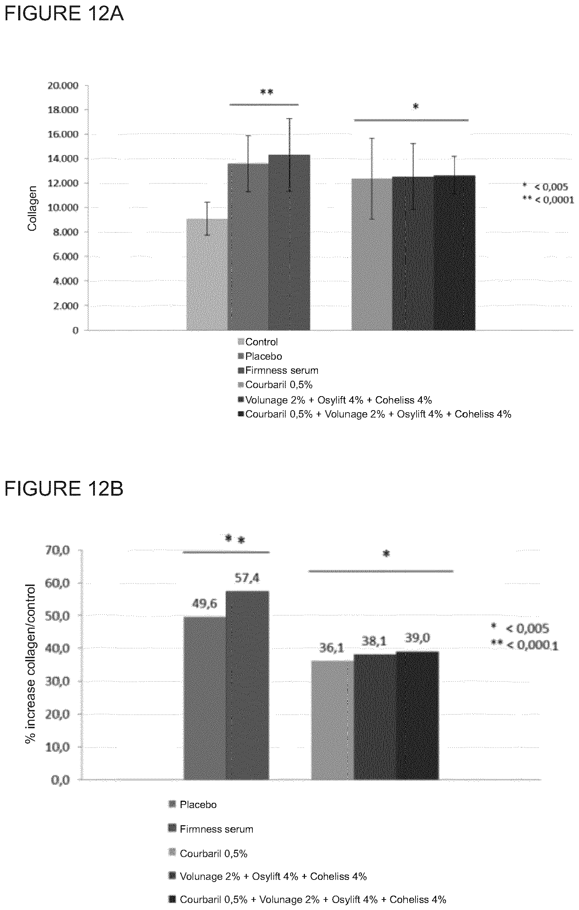

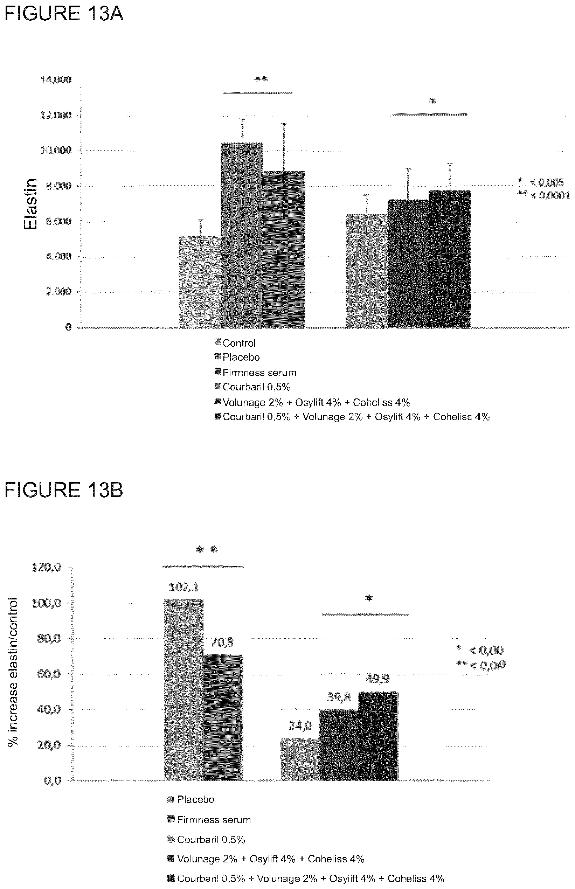

As to the protein expression, the combination of 0.5% Courbaril tree + 2% Volunage + 4% Osilift + 4% Coheliss promoted increases of 39% in the expression of the collagen I protein (Figures 12A and 12B) and 50% in the expression of the elastin protein (Figures 13A and 13B).

### Composition 7 (filler)

The untreated control, placebo, umthe composition cosmética contendo a combination of *Casearia sylvestris, Schinus terebinthfolius* and hyaluronic acid (filler), a combination of *Casearia sylvestris, Schinus terebinthfolius* (1% guaçatonga + 0.025% California pepper tree), 5% hyaluronic acid and a combination of *Casearia sylvestris, Schinus terebinthfolius* and hyaluronic acid (1% guaçatonga + 0.025% California pepper tree + 5% hyaluronic acid) were investigated.

As to the gene expression, as compared to the untreated control, it was seen that the Guaçatonga and California pepper tree sample was able to modulate 153 genes, the Hyaluronic Acid sample was able to modulate 162 genes and the combination of Guaçatonga and California pepper tree and Hyaluronic Acid was able to modulate 144 genes. The combination according to the present invention has been seen to exhibit one-fold effect as compared to the untreated control on claudin I (cell-cell communication mechanism) and on ki-67 (cell proliferation - renewal mechanism), two-fold on elastin (elastic fiber stimulation), SOD2 (endogenous antioxidant system mechanism) and B1 ou B4 integrin (dermis-epidermis cohesion mechanism), four-fold on IL-10 (anti-inflammatory mechanism) and seven-fold relative to involucrin (cell differentiation mechanism).

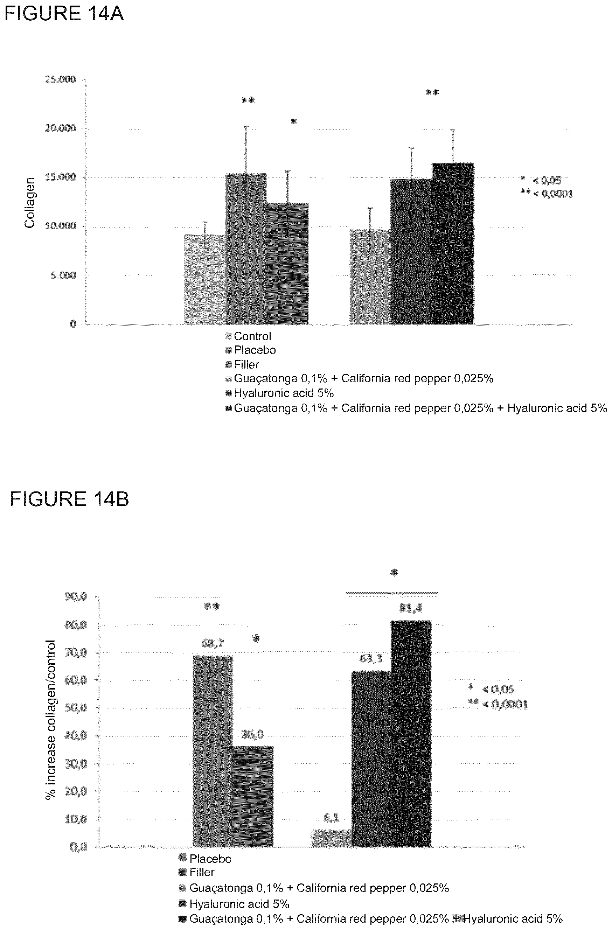

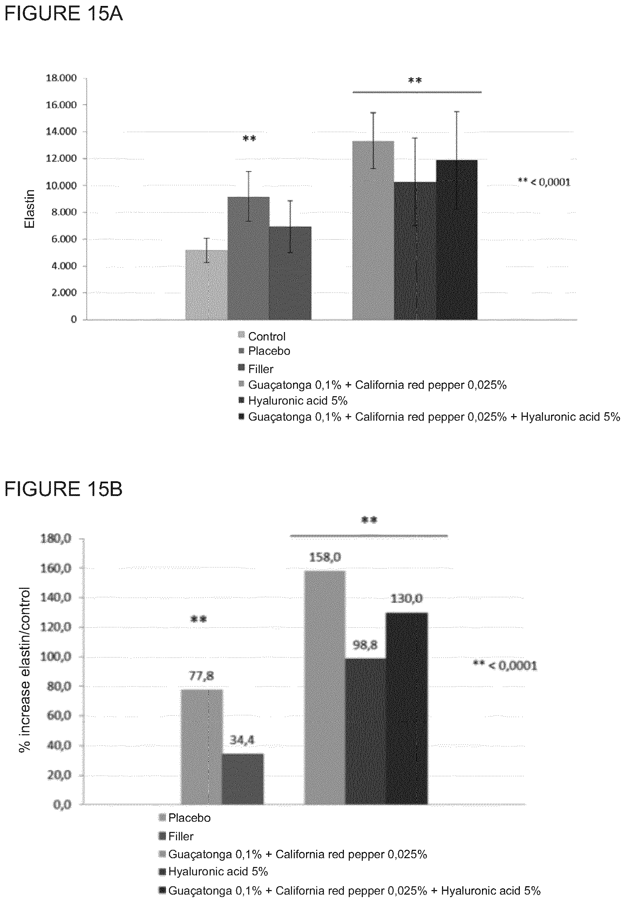

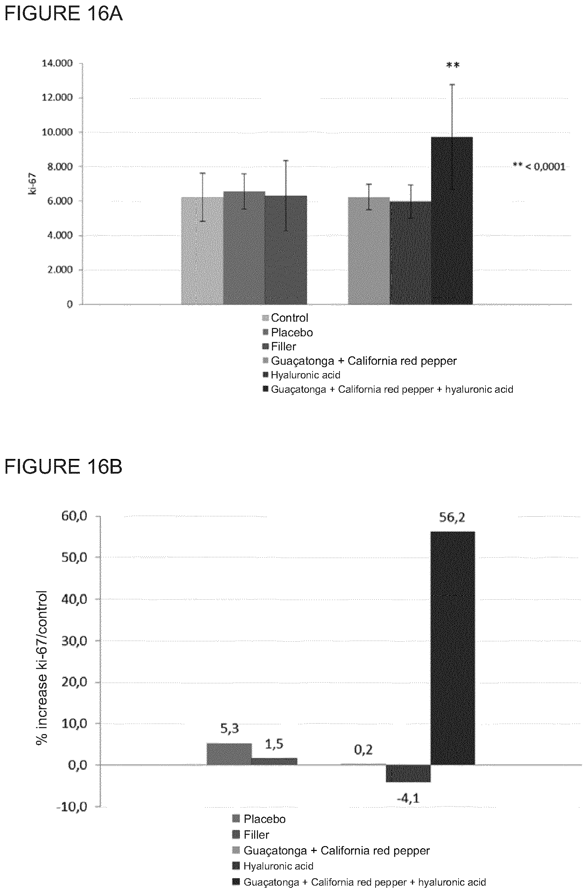

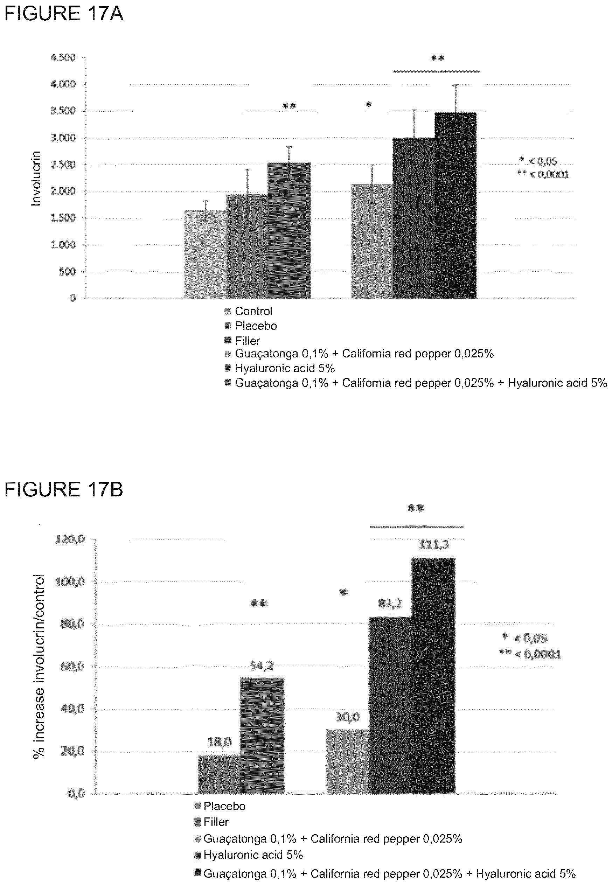

As to the protein expression, the combination of the present invention promoted increases of 31,4% in the expression of the collagen I protein (Figures 14A and 14B), of 130% in the expression of the elastin protein (Figure 15A and 15B) and of 56% in the expression of the ki-67 protein (Figures 16A and 16B). For the involucrin protein, it was seen that the combination according to the present invention promoted a 111,3% increase in the protein expression (Figure 17A and 17B).

### Composition 8 (gel)

The untreated control, placebo, a cosmetic composition containg a combination of *Schinus terebinthfolius* and caffeine (clarifier), *Schinus terebinthfolius* (0.35% California pepper tree), caffeine (1% Ecoslim) and a mixture of *Schinus terebinthfolius* and caffeine (0.35% California pepper tree + 1% Ecoslim) were investigated.

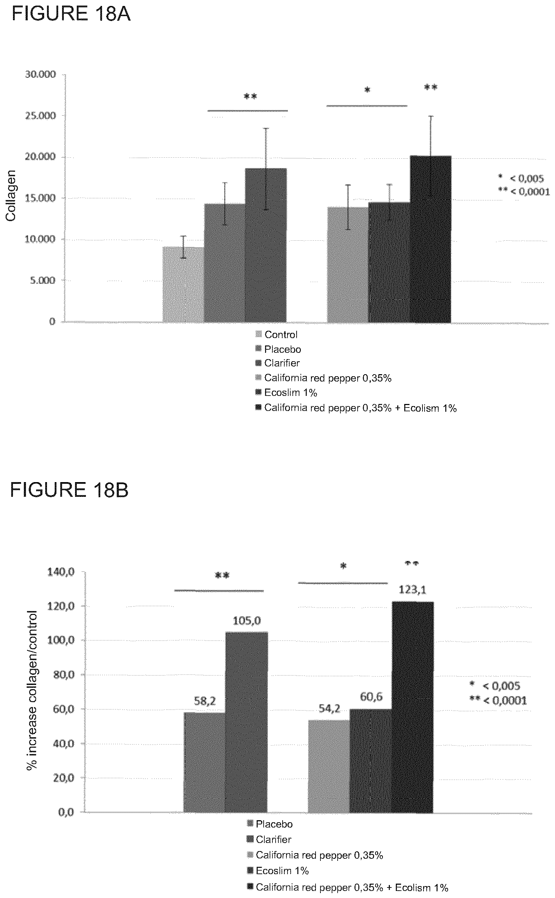

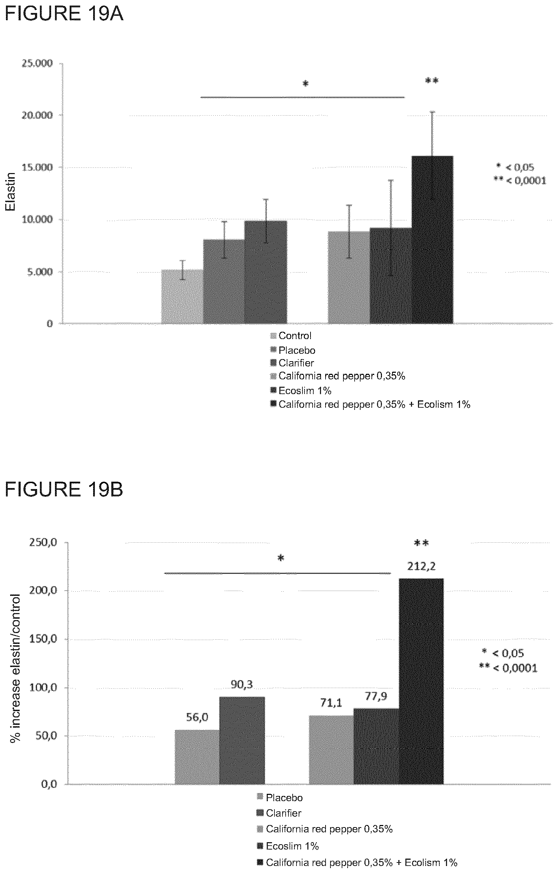

As to the gene expression, as compared to the untreated control, it was seen that the California pepper tree sample was able to modulate 157 genes, the Ecoslim sample was able to modulate 162 genes and the combination of California pepper tree and Ecoslim was able to modulate 160 genes, particularly having a thrice as high performance as compared to the untreated control relative to collagen I (firmness mechanism), four times as high relative to elastin (elastic fiber stimulation), twice as high relative to claudin I (cell-cell communication), four times as high relative to involucrin (cell differentiation), four times as high relative to SOD2 (dermis-epidermis cohesion), eight times as high relative to IL-10 (anti-inflammatory mechanism). As to the protein expression, the combination of California pepper tree and Ecoslim promoted increases of 123% in the expression of the collagen I protein (figure 18A and 18B) and of 212,2% in the expression of the elastin protein (figure 19A and 19B).

Based on the teachings provided in the disclosure of the invention and examples one skilled in the art would be able to appreciate the advantages of the invention and propose variations and alternative equivalent embodiments, without departing from the scope of the invention, as defined in the accompanying claims.

## Claims

1. A COMPOSITION FOR MODULATING GENES RESPONSIBLE FOR THE GENERAL FUNCTIONS OF THE SKIN, **characterized by** comprising at least one plant extract and at least one cosmetically acceptable vehicle.

2. THE COMPOSITION according to claim 1, **characterized in that** said plant extract is selected from the group consisting of *Acmella oleracea (spot-flower), Avena sativa, Camellia sinensis* (green tea), *Casearia sylvestris (guaçatonga), Cichorium intybus* (chicory), *Hymenaea courbaril* (courbaril tree), *Paeonia albiflora* (peony), *Passifloraceae* (passionflower), *Schinus terebinthifolius* (California pepper tree) and *Secale cereal;* either alone or in combination.

3. THE COMPOSITION according to claim 1, **characterized by** further comprising hyaluronic acid, caffeine and/or a mixture of sodium cocoyl amino acids and sarcosine and potassium aspartate and magnesium aspartate.

4. THE COMPOSITION according to one of claims 1 to 3, **characterized by** comprising a combination of at least two components selected from: extracts and/or active ingredients extracted from *Camellia sinensis, Casearia sylvestris, Schinus terebinthifolius, Paeonia albiflora, Cichorium intybus, Hymenaea courbaril, Avena sativa, Secale cereale;* passionflower ceramides; the active ingredients spilanthol and xyloglucan extracted from *Acmella oleracea* and *Hymenaea courbaril,* respectively; hyaluronic acid, caffeine, mistura de sodium cocoyl amino acids and sarcosine and potassium aspartate and magnesium aspartate.

5. THE COMPOSITION according to one of claims 1 to 4, **characterized by** comprising the combination of *Camellia sinensis* extract and spilanthol.

6. THE COMPOSITION according to one of claims 1 to 4, **characterized by** comprising the combination of xyloglucan and a mixture of sodium cocoyl amino acids and sarcosine and potassium aspartate and magnesium aspartate.

7. THE COMPOSITION according to one of claims 1 to 4, **characterized by** comprising the combination of *Casearia sylvestris, Schinus terebinthifolius* and *Paeonia albiflora* extracts.

8. THE COMPOSITION according to one of claims 1 to 4, **characterized by** comprising the combination of passionflower ceramides and *Cichorium intybus* extract.

9. THE COMPOSITION according to one of claims 1 to 4, **characterized by** comprising the combination of spilanthol and hyaluronic acid.

10. THE COMPOSITION according to one of claims 1 to 4, **characterized by** comprising the combination of *Hymenaea courbaril, Paeonia albiflora, Secale cereale* and *Avena sativa* extracts.

11. THE COMPOSITION according to one of claims 1 to 4, **characterized by** comprising the combination of *Casearia sylvestris, Schinus terebinthfolius* extracts, hyaluronic acid.

12. THE COMPOSITION according to one of claims 1 to 4, **characterized by** comprising the combination of *Schinus terebinthfolius* extracts, caffeine and a cosmetically acceptable vehicle.

13. THE COMPOSITION according to claim 5, **characterized by** comprising about 0.025% of *Camellia sinensis* and about 0.125% of spilanthol.

14. THE COMPOSITION according to claim 6, **characterized by** comprising about 0.25% of xyloglucan and about 1.5% of a mixture of sodium cocoyl amino acids and sarcosine and potassium aspartate and magnesium aspartate.

15. THE COMPOSITION according to claim 7, **characterized by** comprising about 0.05% of *Casearia sylvestris,* about 0.0125% of *Schinus terebinthifolius* and about 2% of *Paeonia albiflora.*

16. THE COMPOSITION according to claim 8, **characterized by** comprising about 0.3% of passionflower ceramides and about 3% of *Cichorium intybus.*

17. THE COMPOSITION according to claim 9, **characterized by** comprising about 0.25% of spilanthol and about 5% of hyaluronic acid.

18. THE COMPOSITION according to claim 10, **characterized by** comprising about 0.5% *Hymenaea courbaril,* about 2% of *Paeonia albiflora,* about 4% of *Secale cereale* and about 4% of *Avena sativa.*

19. THE COMPOSITION according to claim 11, **characterized by** comprising de about 0.1% of *Casearia sylvestris,* about 0.025% of *Schinus terebinthfolius* and about 5% of hyaluronic acid.

20. THE COMPOSITION according to claim 12, **characterized by** comprising about 0.35% of *Schinus terebinthfolius* and about 1% of caffeine.

21. THE COMPOSITION according to claim 1, **characterized in that** the cosmetically acceptable vehicles are selected from the group consisting of: preservatives, perfumes/fragrances, polymer neutralizing agents, chelating agents and/or pH adjustment agents.

22. THE COMPOSITION according to claim 1, **characterized in that** the composition is for modulating at least one of the CAT, GPX1, MSRA, NOX1, PRDX6, SOD2, DSC2, IGF1R, ITGA1, ITGB1, LAMB1, LAMB3, DEFB4A, CDH1, CAMP, CLDN1, CLDN4, CLDN7, CDSN, DSG4, DSP, ELOVL3, GBA, ITGA6, ITGB4, KRT19, KRT10. KRT14, KRT16, KRT17, KRT1, KRT6A, OR2AT4, LAMA5, OCLN, PKP1, PLEC, TGM5, TGM1, RNASE7, SMPD1, SDC1, TJP1, VCL, BTC, FLT1, PDGFRA, HAS1, HAS2, SIRT1, SRD5A1, PRLR, HSD17B2, TPH1, AANAT, ASMT, MTNR1A, AR, ESR2, CYP19A1, HTR2A, HTR2B, MTOR, AQP3, CTSB, CTSE, CTSL, CD44, HAL, PADI3, PADI1, CASP14, FLG, ST14, IL13, IL18, IL19, ITGA2, IL1A, IL1B, IL22, IL17A, IL1R1, IL10. IL6, F2RL1, TRPV1, CALCA, ACACA, ASAH1, UGCG, SPTLC1, SREBF2, GLB1, ADAM9, MMP1, MMP10. MMP2, MMP3, MMP9, SERPINE1, TIMP1, TIMP2, TMPRSS6, FBLN5, FBN1, MMP12, MMP13, MMP14, TYR, GPNMB, MAP1LC3B, POMC, OPRM1, MC1R, MITF, CANX, HSPB1, HSPA1A, EGFR, TGFB1, TGFBR1, NGF, PDGFA, VEGFA, VEGFB, FGFR1, FGFR2, FGF1, FGF2, MMP11, HYAL1, HYAL2, ELN, LOX, COL1A1, COL1A2, ACO2, ANXA5 genes.

23. THE COMPOSITION according to one of claims 1 to 22, **characterized in that** the composition is for modulating the expression of at least one of the following proteins involucrin, ki-67, collagen, collagen I, elastin, hyaluronic acid, claudin I.

24. THE COMPOSITION according to one of claims 1 to 23, **characterized in that** the composition is for cell differentiation or proliferation.

25. A METHOD FOR MODULATING THE EXPRESSION OF GENES RESPONSIBLE FOR THE GENERAL FUNCTIONS OF THE SKIN, **characterized by** comprising the step of administering a composition as defined in one of claims 1 to 21 to an individual skin.

26. THE METHOD according to claim 25, **characterized in that** the composition is for modulating at least one of the CAT, GPX1, MSRA, NOX1, PRDX6, SOD2, DSC2, IGF1R, ITGA1, ITGB1, LAMB1, LAMB3, DEFB4A, CDH1, CAMP, CLDN1, CLDN4, CLDN7, CDSN, DSG4, DSP, ELOVL3, GBA, ITGA6, ITGB4, KRT19, KRT10. KRT14, KRT16, KRT17, KRT1, KRT6A, OR2AT4, LAMA5, OCLN, PKP1, PLEC, TGM5, TGM1, RNASE7, SMPD1, SDC1, TJP1, VCL, BTC, FLT1, PDGFRA, HAS1, HAS2, SIRT1, SRD5A1, PRLR, HSD17B2, TPH1, AANAT, ASMT, MTNR1A, AR, ESR2, CYP19A1, HTR2A, HTR2B, MTOR, AQP3, CTSB, CTSE, CTSL, CD44, HAL, PADI3, PADI1, CASP14, FLG, ST14, IL13, IL18, IL19, ITGA2, IL1A, IL1B, IL22, IL17A, IL1R1, IL10. IL6, F2RL1, TRPV1, CALCA, ACACA, ASAH1, UGCG, SPTLC1, SREBF2, GLB1, ADAM9, MMP1, MMP10. MMP2, MMP3, MMP9, SERPINE1, TIMP1, TIMP2, TMPRSS6, FBLN5, FBN1, MMP12, MMP13, MMP14, TYR, GPNMB, MAP1LC3B, POMC, OPRM1, MC1R, MITF, CANX, HSPB1, HSPA1A, EGFR, TGFB1, TGFBR1, NGF, PDGFA, VEGFA, VEGFB, FGFR1, FGFR2, FGF1, FGF2, MMP11, HYAL1, HYAL2, ELN, LOX, COL1A1, COL1A2, ACO2, ANXA5 genes.

27. USE OF A PLANT EXTRACT, **characterized by** being for the preparation of a composition for modulating genes responsible for the general functions of the skin.

28. THE USE according to claim 27, **characterized in that** said plant extract is selected from the group consisting of *Acmella oleracea (spot-flower), Avena sativa, Camellia sinensis* (green tea), *Casearia sylvestris (guaçatonga), Cichorium intybus* (chicory), *Hymenaea courbaril* (courbaril tree), *Paeonia albiflora* (peony), *Passifloraceae* (passionflower). *Schinus terebinthifolius* (California pepper tree) and *Secale cereal;* either alone or in combination.

29. THE USE according to claim 28, **characterized in that** said extract is used in combination with hyaluronic acid, caffeine and/or a mixture of sodium cocoyl amino acids and sarcosine and potassium aspartate and magnesium aspartate.

30. THE USE according to claim 29, **characterized in that** a combination is used of at least two components selected from: extracts and/or active ingredients extracted from *Camellia sinensis, Casearia sylvestris, Schinus terebinthifolius, Paeonia albiflora, Cichorium intybus, Hymenaea courbaril, Avena sativa, Secale cereale;* passionflower ceramides; the active ingredients spilanthol and xyloglucan extracted from *Acmella oleracea* and *Hymenaea courbaril,* respectively; hyaluronic acid, caffeine, a mixture of de sodium cocoyl amino acids and sarcosine and potassium aspartate and magnesium aspartate.

31. THE USE according to claim 27, **characterized in that** the genes are selected from at least one of CAT, GPX1, MSRA, NOX1, PRDX6, SOD2, DSC2, IGF1R, ITGA1, ITGB1, LAMB1, LAMB3, DEFB4A, CDH1, CAMP, CLDN1, CLDN4, CLDN7, CDSN, DSG4, DSP, ELOVL3, GBA, ITGA6, ITGB4, KRT19, KRT10. KRT14, KRT16, KRT17, KRT1, KRT6A, OR2AT4, LAMA5, OCLN, PKP1, PLEC, TGM5, TGM1, RNASE7, SMPD1, SDC1, TJP1, VCL, BTC, FLT1, PDGFRA, HAS1, HAS2, SIRT1, SRD5A1, PRLR, HSD17B2, TPH1, AANAT, ASMT, MTNR1A, AR, ESR2, CYP19A1, HTR2A, HTR2B, MTOR, AQP3, CTSB, CTSE, CTSL, CD44, HAL, PADI3, PADI1, CASP14, FLG, ST14, IL13, IL18, IL19, ITGA2, IL1A, IL1B, IL22, IL17A, IL1R1, IL10. IL6, F2RL1, TRPV1, CALCA, ACACA, ASAH1, UGCG, SPTLC1, SREBF2, GLB1, ADAM9, MMP1, MMP10. MMP2, MMP3, MMP9, SERPINE1, TIMP1, TIMP2, TMPRSS6, FBLN5, FBN1, MMP12, MMP13, MMP14, TYR, GPNMB, MAP1LC3B, POMC, OPRM1, MC1R, MITF, CANX, HSPB1, HSPA1A, EGFR, TGFB1, TGFBR1, NGF, PDGFA, VEGFA, VEGFB, FGFR1, FGFR2, FGF1, FGF2, MMP11, HYAL1, HYAL2, ELN, LOX, COL1A1, COL1A2, ACO2, ANXA5.
